# EUROPEAN PATENT SPECIFICATION

(11) **EP 1 487 467 B1**
(45) Date of publication and mention of the grant of the patent: **29.11.2006**
(21) Application number: 02717004.2
(22) Date of filing: 26.03.2002
(51) Int. Cl.: A61K 36/899, A61P 31/04

(54) **BIOACTIVE HEXANE FRACTION FROM VETIVERIA ZIZANIOIDES**
BIOAKTIVE HEXAN FRAKTION AUS VETIVERIA ZIZANIOIDES
FRACTION D'HEXANE BIOACTIVE DE VETIVERIA ZIZANIOIDES

(43) Date of publication of application: 22.12.2004
(73) Proprietor: COUNCIL OF SCIENTIFIC AND INDUSTRIAL RESEARCH, New Delhi 110 001 (IN)
(72) Inventor: KHANUJA, Suman, Preet, Singh, Uttar Pradesh 226 015 (IN); SRIVASTAVA, Suchi, Uttar Pradesh 226 015 (IN); KUMAR, Tiruppadiripuliyur, Ranganathan, Santha, Uttar Pradesh 226 015 (IN); GUPTA, Madan, Mohan, Uttar Pradesh 226 015 (IN); TRIPATHY, Arvind, Kumar, Uttar Pradesh 226 015 (IN); SINGH, Monika, Uttar Pradesh 226 015 (IN); BAHL, Janak, Raj, Uttar Pradesh 226 015 (IN); LAL, Raj, Kishori, Uttar Pradesh 226 015 (IN); DAROKAR, Mahendra, Pandurang, Uttar Pradesh 226 015 (IN); SHASANY, Ajit, Kumar, Uttar Pradesh 226 015 (IN); KUMAR, Sushil, Uttar Pradesh 226 015 (IN)
(74) Representative: Nargolwalla, Cyra
(86) International application number: PCT/IB2002/001157
(87) International publication number: WO 2003/080089

(56) References cited:
- DATABASE BIOSIS [Online] BIOSCIENCES INFORMATION SERVICE, PHILADELPHIA, PA, US; 1990 GANGRADE S K ET AL: "EVALUATION OF SOME ESSENTIAL OILS FOR ANTIBACTERIAL PROPERTIES" Database accession no. PREV199191099114 XP002219889 & INDIAN PERFUMER, vol. 34, no. 3, 1990, pages 204-208, ISSN: 0019-607X
- HAMMER K A ET AL: "ANTIMICROBIAL ACTIVITY OF ESSENTIAL OILS AND OTHER PLANT EXTRACTS" JOURNAL OF APPLIED MICROBIOLOGY, OXFORD, GB, vol. 86, no. 6, 1999, pages 985-990, XP001108841 ISSN: 1364-5072

## Description

### FIELD OF THE INVENTION

The present invention relates to a hexane bioactive fraction and obtained from the roots of an aromatic plant named *Vetiveria zizanioides* commonly found in India for inhibiting the growth of drug resistant bacterial infections in humans and animals. The invention also relates to a pharmaceutical composition comprising the bioactive extract with other additives for inhibiting the growth of drug resistant bacterial infections in humans and animals. The present invention also provides a process for the isolation of said bioactive extract.

### BACKGROUND OF INVENTION

Antibiotics have been used for long to cure bacterial, fungal and other infectious diseases of humans. Penicillin was the first antibiotic used against infections during the Second World War. Since then a number of antibiotics and their derivatives have been identified and used by man almost all of which were isolated from microbial sources. All the antibiotics in clinical use today can be grouped or classified according to their structure or functional groups. Streptomycin, kanamycin, tetracycline some of the well-known examples are aminoglycosides whereas penicillin and its derivatives are beta-lactam antibiotics. One of the commonly used antibacterials are quinolones or fluoroquinolones such as nalidixic acid, ciprofloxacin, norfloxacin etc. Fluoroquinolones are now widely used to treat urinary tract infections, upper respiratory tract infections, and tuberculosis, which are resistant to first-line drugs. However, many of the pathogenic bacteria such as *Haemophilus influenzae, Neisseria Sp., Staphylococcus aureus, Escherichia coli* are developing resistance to fluoroquinolone class of antibiotics limiting their clinical usefulness. Since, the mechanism of action of all the quinolones against bacteria is similar, development of resistance to one of the quinolone antibiotic would confer simultaneous cross-resistance to almost all the other quinolones also. Fluoroquinolones act by inhibiting the function of a bacterial enzyme DNA gyrase essential for the maintenance of supercoil nature of the bacterial chromosome. Resistance development is observed when a mutation in the DNA gyrase enzyme A subunit (GyrA+) specifically in the region called "Quinolone Determining Region (QDR)" occurs. The modified mutant form of A subunit (GyrA-) is incapable of binding to quinolone antibiotics and therefore is resistant. Such quinolone resistant infections are particularly difficult to cure. Kumar et al (*Phytotherapy Research 14: 14-15, 2000; US Patent No.6, 127,405)* have identified a semi-synthetic plant compound α -arteether which is capable of specifically killing quinolone drug resistant bacterial infections. The α-arteether was obtained by etherification of artemissinin a sesquiterpene lactone compound from a Chinese medicinal plant *Artemisia annua.* In our effort to isolate and identify more potent plant compounds which are active against quinolone resistant bacteria we carried out a systematic bioactivity guided fractionation of the ethanolic extract prepared from the roots of Indian medicinal plant *Vetiveria zizanioides.* The subject mentioned below specifically describes the manner in which the compound inhibiting quinolone resistant bacteria was isolated and identified.

### Objects of the invention

The main object of the invention is to develop a novel anti bacterial agent inhibiting the growth of multi drug resistant bacterial pathogens.

Another object of the invention is to provide a bioactive fraction from the roots of plant *Vetiveria zizanioides.*

Another object of the invention is to provide a pharmaceutical composition comprising bioactive fraction or plant extract obtained from plant *Vetiveria zizanioides* Still another object of the invention is to provide a method of isolation of bioactive fraction from the roots of plant *Vetiveria zizanioides.*

### SUMMARY OF THE INVENTION

Accordingly, the present invention provides a bioactive hexane fraction named as CIM 109 obtained from the roots of plant *Vetiveria zizanioides* for inhibiting growth of multidrug resistant bacterial pathogens. The present invention also provides a pharmaceutical composition comprising bioactive fraction CIM 109 or plant extract or lyophilised extract to provide anti bacterial activity.

### DETAILED DESCRIPTION OF INVENTION

Accordingly, the present invention provides a bioactive hexane fraction CIM 109 obtained from the plant *Vetiveria zizaniodes* having inhibitory activity against multi drug resistant bacterial pathogens.

In one embodiment of the invention, the said bioactive fraction is useful to inhibit the growth of bacterial pathogens which are resistant to nalidixic acid, oxolinic acid, sparfloxicin, ciprofloxicin, lomefloxicin and any other quinolones.

In another embodiment of the invention, the multidrug resistant bacteria is selected from the group consisting of genus *Mycobacterium* or *Escherchia coli* preferably selected from group consisting of *Mucobacterium smegmatis MG*^{*2*} *155, Pseudomonas aeruginosa, Bacillus subtilis MTCC-121, Mucobacterium smegmatis MC*^{*2*} *155 Wld type. Mucobacterium smegmatis MC*^{*2*} *155 (NaiR) 6b, Mucobacterium smegmatis MC*^{*2*} *155 13a and E.Coli DH5a.*

One more embodiment of the invention relates to a pharmaceutical composition for inhibiting the growth of the bacterial pathogens, comprising effective amount of bioactive fraction named CIM-109 or partially purified extract or lyophilised extract, obtained from the plant *Vetiveria zizaniodes.*

In another embodiment of the invention, the composition containing the said bioactive fraction is used singly or in combination thereof to the patient.

In still another embodiment, the composition may be administered systematically or orally and preferably orally.

In still another embodiment, the bioactive fraction is administered to the patient in combination with a pharmaceutically acceptable additives carriers, diluent, solvent, filter, lubricant, excipient, binder or stabiliser.

Yet another embodiment relates to the additive used which is selected from a group consisting of citric acid, calcium carbonate, magnesium hydroxide gel and/or gel and/or lactose.

Yet another embodiment relates to amount of active fraction in the composition in the range of 100 mg to 500 mg.

Yet another embodiment of the invention relates to amount of composition administered to a subject in the range of 500 mg to 1000 mg per day.

Yet another embodiment of the invention relates to amount of composition administered to a subject preferably in the range of 150 mg to 700 mg per day.

In yet another embodiment of the invention, the subject is selected mammals, animals preferably humans.

Another embodiment of the invention provides a pharmaceutical composition useful for treating fluoroquinolone resistant bacterial infections including enteric and systemic infections, said composition comprising 10 to 50% by wt of root extract of vetiver, 0.4 to 1 % by wt of citric acid, 10 to 20% by wt of calcium carbonate, 10 to 20% by wt of magnesium hydroxide gel, 20 to 60 % by weight of lactose and optionally comprising other pharmaceutically acceptable additives. The above said composition can optionally be compounded with honey by dispersing the constituents in honey.

One more embodiment of the invention relates to the use of a bioactive hexane fraction or a lyophilised bioactive hexane fraction from the roots of plant *Vetiveria zizaniodes* for the reparation of a medicament for the treatment of a bacterial infection caused by fluoroquinolone resistant bacterial infections and multidrug resistant bacteria infections including enteric and systemic infections.

Another embodiment of the invention relates to a process for the isolation of bioactive fraction from the plant *Vetiveria zizaniodes* having inhibitory activity against multi drug resistant bacterial pathogens, the said process comprises steps of:
a) powdering the plant part of *Vetivera Zizanioides,*
b) extracting the plant powder of step (a) by soaking in protic aqueous organic solvent for a period of 16-20 hours,
c) filtering the organic solvent extract of step (b),
d) evaporating the extract of step (c) under reduced pressure to remove the organic solvent to obtain an aqueous extract,
e) lyophilising the aqueous extract of step (d) to get an powdered extract,
f) dissolving the powdered extract of step (e) in 2% aqueous citric acid,
g) extracting the solution of step (f) successively with chloroform, n-butanol, methanol and finally with acetone to obtain respective organic extracts and an aqueous solution,
h) evaporating separately the organic extracts of step (g) to obtain respective residues,
i) neutralizing the aqueous solution of step (g) with ammonia solution,
j) testing the bioactivity of residues obtained in step (h) and neutralized solution of step (i) to identify residue from methanolic extract as bioactive residue,
k) macerating the residue of methanoloic extract of step (h) successively with hexane, chloroform and ethylacetate,
l) testing bioactivity of hexane, chloroform and ethylacetate fractions of step (k) to identify hexane fractions as a bioactive fraction,
m) purifying the residue of hexane fraction of step (l) on a silica gel column using eluant hexane and mixture of hexane-chloroform with increasing polarity, and
n) evaporating the hexane- chloroform (1:1) eluant fraction obtained from step (m) to yield a residue, which is purified by thin layer chromatography to achieve the required bioactive fraction.

Another embodiment of the invention, in which the bioactive fraction obtained in step (m), is designated as CIM 109.

Still another embodiment of the invention provides a bioactive fraction obtained from roots of *Vetivera Zizanioides.*

In still another embodiment of the invention, the protic aqueous organic solvent used in step (b) is selected from aqueous alcohol preferably aqueous ethanol.

In our study specifically directed at finding an antibiotic or plant compound, which can specifically kill quinolone drug resistant bacterial infections, we found that the ethanolic extract of roots of *Vetiveria zizanioides* was able to kill GyrA- mutant *E.coli* bacteria but not the wild type strains (GyrA+). The ethanolic extract was then fractionated by liquid-liquid chromatography and the hexane fraction was found to possess the growth inhibitory activity. The hexane fraction was then fractionated using silica gel column chromatography wherein the fraction eluted using 10% Chloroform in hexane indicated the desired bioactivity of eliminating *Mycobacterium smegmatis* (GyrA-) growth. Further to isolate and purify the active principle the eluted 10% chloroform in hexane fraction was separated by column chromatography. The Chl:Hex (50:50) fraction obtained from this column was able to kill the GyrA- strains of *E.coli* and *M.smegmatis* bacteria. The Chl:Hex (50:50) fraction was then purified by thin layer chromatography (TLC) to obtain a pure fraction called CIM 109 which has shown the desired bioactivty. Thus CIM 109 isolated from the roots of *Vetiveria zizanioides* was able to inhibit the growth of fluoro-quinolone resistant (GyrA-) bacteria. Hence, CIM 109 can be used for treating quinolone resistant bacterial infections of human and animals.

The detailed description of the invention is explained in the form of examples and should not construed to limit the scope of the invention.

### Example-1

The roots of *Vetiveria zizanioides* genotype KS-1 grown in CIMAP farm harvested during the month of April was dried in shade and ground to fine powder and then extracted by dipping the plant material overnight in different solvents. The solvent was then evaporated in vacuo and the residue termed as root extracts was redissolved in dimethylsulfoxide (DMSO) at the required concentration and analysed for bioactivity.

### Example-2

The ethanolic extracted prepared as above was tested for their growth inhibitory property against a number of bacterial strains by disc diffusion assay a procedure commonly followed and can be performed by persons skilled in the art. The results indicated that the ethanolic extracted residue showed a surprisingly interesting inhibitory activity against many bacterial strains. Hence, to explore the potential of using the extracts as anti-microbial agents the ethanolic extracted was then fractionated using different solvents as follows:

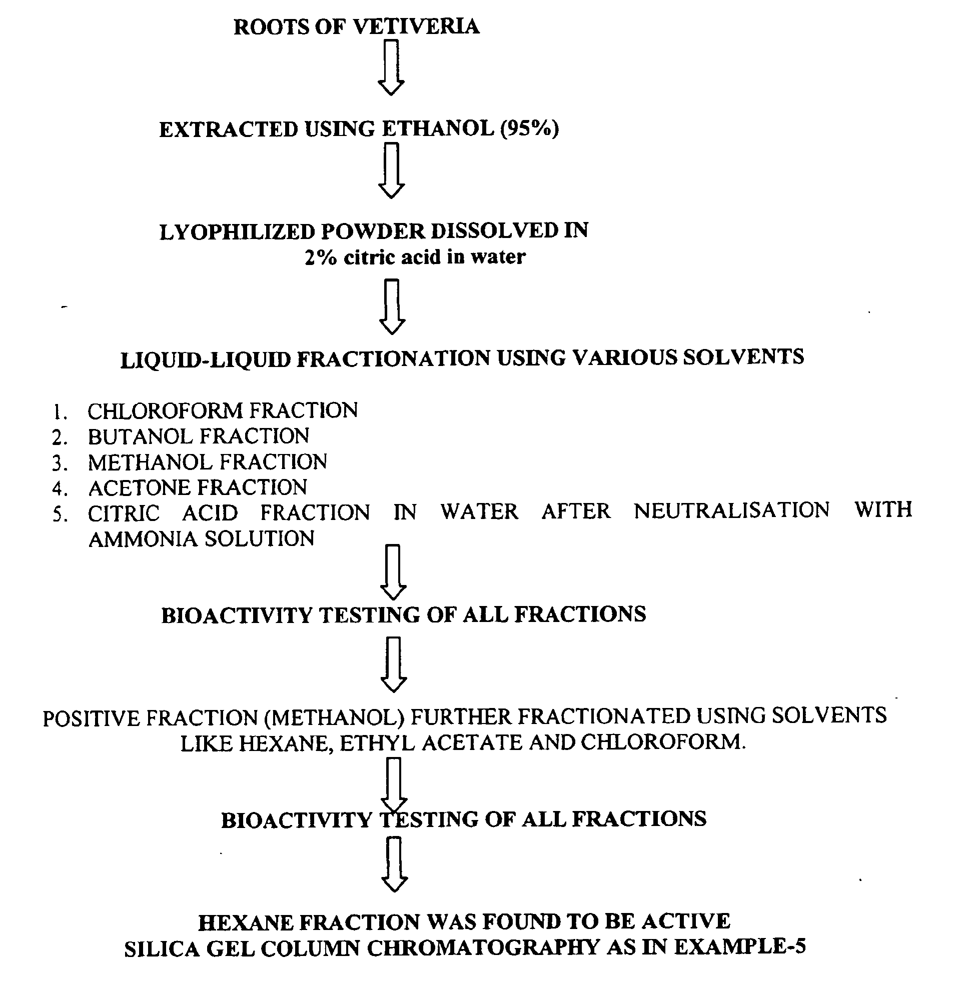

### Example-3

The ethanolic extract of the roots of *Vetiveria zizanioides* was further fractionated by liquid - liquid chromatography using different solvents. The extracts were initially dissolved in citric acid containing water and the solvent extraction was performed with increasing degree of polarity. The solvent extracts obtained were then evaporated in vacuo and the residues were analysed for their anti-microbial activity by disc diffusion assays. The results as in table-1 indicate that the methanolic fraction exhibited maximum bioactivity against bacterial pathogens. We observed an interesting feature that the methanolic fraction does not inhibit the growth of wild type *E.coli* starin CA8000 but was inhibitory to the nalidixic acid resistant strains of *E.coli* DH5a, NK5819 and ET8000. These strains were resistant to nalidixic acid by virtue of carrying mutations in the *gyr*A gene, which also confers resistance to other fluoroquinolones (FQ) such as ciprofloxacin, norfloxacin, levofloxacin etc. Hence we hypothesised that the methanolic extract might contain some active principle, which is able to specifically kill the FQ resistant bacteria but not the normal sensitive bacteria such as CA8000. Therefore to isolate the biologically active principle fraction, the applicants resorted to column chromatography of the methanolic fraction of the ethanolic root extract of *Vetiveria zizanioides* KS-1.

**Table-1: Bioactivity Response of different solvent fractions of the root extracts of Vetiveria zizanioides on different Bacterial Strains added @ 0.8mg/disc.**

| Strains | Zone of growth inhibition (mm) | | |
|---|---|---|---|
| | Methanol | Spirit | Citric Acid |
| *Salmonella typhimurium* | 2 | 2 | - |
| *Mycobacterium smegmatis* MC² 155 | 4 | 2 | 1 |
| *Pseudomonas aeruginosa* | 7 | 6 | 9 |
| *Bacillus subtilis* MTCC-121 | 1 | | |
| *E.coli* CA8000 | - | - | - |
| *E.coli* DH5□ | 2 | - | - |
| *E.coli* NK5819 | 2 - | | - |
| *E.coli* ET8000 | 2 | - | - |

### Example-4

Further to test whether the active principle present in the methanolic can inhibit the growth of *Mycobacterium* sp. we fractionated the methanolic extract using three different solvents by liquid - liquid chromatography a commonly used technique which a person skilled in the art can perform. The bioactivity testing against *Mycobacterium smegmatis* strain Mc²155 results showed that the hexane, chloroform and methanol fractions were inhibitory to the nalidixic acid resistant strains (NaIR) (6b and 13a) of *Mycobacterium smegmatis* strain Mc²155 but not the wild type.

**Table-2: Bioactivity response (zone of growth inhibition) of liquid-liquid fractions of the methanolic extract of Vetiveria zizanioides KS-1 on M. smegmatis.**

| | Mycobacterium smegmatis *strain Mc*^{*2*}*155* (wld type) | *Mycobacterium smegmatis* strain Mc²155 (NalR) 6b | *Mycobacterium smegmatis* strain Mc²155 13a |
|---|---|---|---|
| Hex fraction | - | 10 mm | 4 mm |
| Ethyl acetate | - | - | - |
| Chloroform | - | 5 mm | 3 mm |
| Meth. Fraction | - | 6 mm | 15 mm |

Hence, it is clear from the above observation that the active principle not only inhibits the growth of FQ resistant *E.coli* strains as in example-3 but also inhibits the growth of the FQ resistant *Mycobacterium smegmatis* strain 6b and 13a specifically.

### Example-5

Further to purify the active principle silica gel column chromatography was performed. Any person who is skilled in the art of related subjects can perform the technique. About 20g of hexane fraction was packed onto a silica gel column and the fractions were eluted in hexane - chloroform solvent system. The table-3 below gives the number of column fractions collected using each solvent system. The column fractions were subsequently analysed for their inhibitory activity against *M.smegmatis* wild type and the mutants resistant to nalidixic acid. The results indicate that the column fractions eluted using 10% Hexane in chloroform was able to inhibit only the nalidixic acid resistant strains of *M.smegmatis* and not the wild type bacteria whereas the 15% and 25% Hexane in chloroform was able to inhibit both the wild type and Nal R mutant indicating that it is non specific. Moreover the 60% Hexane: Chloroform fraction was able to inhibit the NalR strains of *E.coli* but not the wild type. Therefore the 10-60% hexane in chloroform fraction can inhibit the fluoroquinolone resistant strains of both *E.coli* and *M.smegmatis* and therefore possess the ability to cure such drug resistant infections in humans and animals.

| Solvent system used | Amount of each fraction collected | No. of fractions collected | No. of fractions collected |
|---|---|---|---|
| Hexane | 100 ml | 20 | 2 lit. |
| 10% CHCl₃ in Hexane | 100 ml | 150 | 15 lit. |
| 15% CnCl₃ in Hexane | 100 ml | 15x5=75 | 7.5 lit. |
| 25% CHCl₃ in Hexane | 100 ml | 16x4=64 | 8 lit. |
| 30% CHCl₃ in Hexane | 100 ml | 8x4=32 | 4 lit. |
| 35% CHCl₃ in Hexane | 100 ml | 10x4=40 | 5 lit. |
| 40 % CHCl₃ in Hexane | 100 ml | 10x4=40 | 5 lit. |
| 50% CHCl₃ in Hexane | 100 ml | 6x4=24 | 3 lit. |
| 75% CHCl₃ in Hexane | 100 ml | 16x4=64 | 8 lit. |
| CHCl₃ fraction | 100 ml | 13x4=52 | 7 lit. |
| 5% Eto Ac | 100 ml | 8x4=32 | 4 lit. |
| 10% Eto Ac | 100 ml | 6x4=24 | 3 lit. |
| 20% Eto Ac | 100 ml | 4x4=16 | 2 lit. |
| 30% Eto Ac | 100 ml | 4x4=16 | 2 lit. |
| 50% Eto Ac | 100 ml | 4x4=16 | 2 lit. |

**Bioactivity profile of various column chromatography fractions against *M.smegmatis* and the Nal R mutant 6b.**

| Fraction No. | Solvent system used for the elution | Range of Zone of growth inhibition (400mcg/disc) | | |
|---|---|---|---|---|
| | | *M.smegmatis* | | *E.coli* DH5a |
| | | (wild type) | 6b (nalR) | |
| 0 1-6 | 10% Hex.:Chl. | - | - | - |
| OY-6 | 25% Hex.:Chl. | 3-5 | 5-10 | - |
| OY-11 | 15% Hex.:Chl. | 2-5 | 7-24 | - |
| LY-11 | 30% Hex.:Chl. | 2-5 | 2-18 | 3-4 |
| | 60% Hex: Chl | | 2-10 | |
| CIM-109 | TLC purified fraction | | 12-15 | 6- 8 |

## Claims

1. A process for the isolation of bioactive fraction comprising the steps of :
a) powdering the plan part of Vetivera zizanioides,
b) extracting the plant powder of step (a) by soaking in protic aqueous organic solvent for a period of 16-20 hours,
c) filtering the organic solvent extract of step (b),
d) evaporating the extract of step (c) under reduced pressure to remove the organic solvent to obtain an aqueous extract,
e) lyophilising the aqueous extract of step (d) to get an powdered extract,
f) dissolving the powdered extract of step (e) in 2% aqueous citric acid,
g) extracting the solution of step (f) successively with chloroform, n-butanol, methanol and finally with acetone to obtain respective organic extracts and an aqueous solution,
h) evaporating separately the organic extracts of step (g) to obtain respective residues,
i) neutralizing the aqueous solution of step (g) with ammonia solution,
j) testing the bioactivity of residues obtained in step (h) and neutralized solution of step (i) to identify residue from methanolic extract as bioactive residue,
k) macerating the residue of methanoloic extract of step (h) successively with hexane, chloroform and ethylacetate,
l) testing bioactivity of hexane, chloroform and ethylacetate fractions of step (k) to identify hexane fractions as a bioactive fraction,
m) purifying the residue of hexane fraction of step (l) on a silica gel column using eluant hexane and mixture of hexane-chloroform with increasing polarity, and
n) evaporating the hexane-chloroform (1:1) eluant fraction obtained from step (m) to yield a residue, which is purified by thin layer chromatography to achieve the required bioactive fraction.

2. The process as claimed in claim 1, wherein the bioactive fraction obtained in step (m) is designated as CIM 109.

3. The process as claimed in claim 1, wherein the plant part is selected from roots of *Vetivera zizanioides.*

4. The process as claimed in claim 1, wherein in step (b) the protic aqueous organic solvent is aqueous ethanol.

5. A bioactive hexane fraction CIM 109 from the plant *Vetivera zizanioides* having inhibitory activity against multi drug resistant bacterial pathogens, wherein the fraction is obtainable from step (m) of the process as claimed in claims 1 to 4.

6. The bioactive fraction as claimed in claim 5, said bioactive fraction inhibits the growth of bacterial pathogens which are resistant to nalidixic acid, oxolinic acid, sparfloxicin, ciprofloxacin, lomefloxicin and any other quinolones.

7. The bioactive fraction as claimed in claim 6, wherein multidrug resistant bacteria is selected from the group consisting of genus *Mycobacterium* or *Escherichia coli* preferably selected from group consisting of *Mucobacterium smegma tis Mc*^{*2*} *155, Pseudomonas aeruginosa, Bacillus subtilis MTCC-121, Mucobacterium smegmatis MC*^{*2*} *155 Wld type, Mucobacterium smegmatis MC*^{*2*} *155 (NaiR) 6b, Mucobacterium smegmatis MC*^{*2*} *155 13a and E. Coli DH5a.*

8. A pharmaceutical composition for inhibiting the growth of the bacterial pathogens, this said composition comprising effective amount of bioactive fraction CIM-109 or partially purified extract or lyophilised extract of CIM-109 as claimed in claim 5.

9. The composition as claimed in claim 8, wherein said bioactive fraction is used singly or in combination thereof .

10. The composition as claimed in claim 8, wherein said bioactive fraction may be administered systematically or orally preferably orally.

11. The composition as claimed in claim 8, wherein the bioactive fraction is to be administered to the patient in combination with pharmaceutically acceptable additives, carriers, diluent, solvent, filter, lubricant, excipient, binder or stabiliser.

12. The composition as claimed in claim 8, wherein the additive used is selected from a group consisting of citric acid, calcium carbonate, magnesium hydroxide gel or gel or lactose.

13. The composition as claimed in claim 8,wherein the daily dosage for humans is in the range of 500 mg to 1000 mg.

14. The composition as claimed in claim 8, wherein the amount of bioactive fraction in the composition is in the range of 100 mg to 500 mg.

15. A pharmaceutical composition useful for treating fluoroquinolone resistant bacterial infections and inhibiting multidrug resistant bacterial infections including enteric and systemic infections, said composition comprising 10 to 50% by wt of bioactive hexane fraction from root extract of vetiver, 0.4 to 1% by wt of citric acid, 10 to 20% by wt of calcium carbonate, 10 to 20% by wt of magnesium hydroxide gel, 20 to 60% by weight of lactose and optionally along with other pharmaceutically acceptable additives.

16. The pharmaceutical composition as claimed in claim 15 is optionally compounded with honey by dispersing the constituents in honey.

17. A use of a bioactive hexane fraction or a lyophilised bioactive hexane fraction from the roots of plant *Vetiveria zizaniodes* for the preparation of a medicament for the treatment of bacterial infections caused by fluoroquinolone resistant bacterial infections and multidrug resistant bacteria infections including enteric and systemic infections.

18. The use as claimed in claim 17, wherein said hexane bioactive fraction is used singly or in combination thereof .

19. The use as claimed in claim 17, wherein said medicament is suitable for a systemic or oral and preferably oral administration.

20. The use as claimed in claim 17, wherein said medicament further comprises pharmaceutically acceptable additives, carriers, diluent, solvent, filter, lubricant, excipient, binder or stabiliser,

21. The use as claimed in claim 17, wherein the additive used is selected from a group consisting of citric acid, calcium carbonate, magnesium hydroxide gel and/or gel and/or lactose.

22. The use as claimed in claim 17, wherein said medicament is suitable for the daily dosage ranging from 100 mg to 500 mg.

23. The use as claimed in claim 17, wherein said medicament is suitable for the preferred dosage ranging from 150 mg to 250 mg.

## Patentansprüche

1. Verfahren zur Isolierung einer biologisch aktiven Fraktion umfassend die Schritte:
a) Pulverisieren des pflanzlichen Teils von Vetivera zizanioides,
b) Extrahieren des Pflanzenpulvers aus Schritt (a) durch Tränken in einem protischen wässrigen organischen Lösungsmittel für einen Zeitraum von 16 bis 20 Stunden,
c) Filtrieren des organischen Lösungsmittelextraktes aus Schritt (b),
d) Verdampfen des Extrakts aus Schritt (c) unter reduziertem Druck, um das organische Lösungsmittel zu entfernen, um einen wässrigen Extrakt zu erhalten,
e) Lyophilisieren des wässrigen Extrakts aus Schritt (d), um einen gepulverten Extrakt zu bekommen,
f) Lösen des gepulverten Extrakts aus Schritt (e) in 2-prozentiger wässriger Zitronensäure,
g) Extrahieren der Lösung aus Schritt (f) nacheinander mit Chloroform, n-Butanol, Methanol und schließlich mit Azeton, um jeweils organische Extrakte und eine wässrige Lösung zu gewinnen,
h) separates Verdampfen der organischen Extrakte aus Schritt (g), um die entsprechenden Reste zu gewinnen,
i) Neutralisieren der wässrigen Lösung aus Schritt (g) mit Ammoniaklösung,
j) Testen der biologischen Aktivität der Reste, die in Schritt (h) und der neutralisierten Lösung aus Schritt (i) gewonnen wurden, um einen Rest des methanolischen Extrakts als biologisch aktiven Rest zu identifizieren,
k) Mazerieren des Rests des methanolischen Extrakts aus Schritt (h) nacheinander mit Hexan, Chloroform und Ethylazetat,
l) Testen der biologischen Aktivität der Hexan-, Chloroform-, und Ethylacetatfraktionen aus Schritt (k), um Hexanfraktionen als biologisch aktive Fraktionen zu identifizieren,
m) Reinigen des Restes der Hexanfraktion aus Schritt (l) über eine Kieselgelsäule unter Verwendung des Elutionsmittels Hexan und eines Gemischs aus Hexan-Chloroform mit ansteigender Polarität, und
n) Verdampfen des Hexan-Chloroform(1:1)-Elutionsmittelfraktion, die in Schritt (m) gewonnen wurde, um einen Rest zu ergeben, welcher durch Dünnschichtchromatographie gereinigt wird, um die benötigte biologisch aktive Fraktion zu erzielen.

2. Verfahren wie in Anspruch 1 beansprucht, worin die biologisch aktive Fraktion, die in Schritt (m) gewonnen wurde, als CIM-109 bezeichnet wird.

3. Verfahren wie in Anspruch 1 beansprucht, worin der Pflanzenteil aus den Wurzeln von Vetivera zizanioides ausgewählt ist.

4. Verfahren wie in Anspruch 1 beansprucht, worin im Schritt (b) das protische wässrige organische Lösungsmittel wässriger Ethanol ist.

5. Biologisch aktive Hexanfraktion CIM-109 aus der Pflanze Vetivera zizanioides mit inhibitorischer Wirkung gegen Multi-Medikament-resistente Bakterienpathogene, wobei die Fraktion aus Schritt (m) des Verfahrens, wie in den Ansprüchen 1 bis 4 beansprucht, erhältlich ist.

6. Biologisch aktive Fraktion wie in Anspruch 5 beansprucht, wobei diese biologisch aktive Fraktion das Wachstum bakterieller Pathogene inhibiert, die gegen Nalidixinsäure, Oxolinsäure, Sparfloxicin, Ciprofloxacin, Lomefloxicin und alle anderen Chinolone resistent sind.

7. Biologisch aktive Fraktion wie in Anspruch 6 beansprucht, wobei das Multi-Medikament-resistente Bakterium aus der Gruppe ausgewählt ist, die aus dem Genus Mycobacterium oder Escherichia coli besteht, bevorzugt ausgewählt aus der Gruppe, die aus Mucobacterium smegmatis MC² 155, Pseudomonas aeruginosa, Bacillus subtilis MTCC-121, Mucobacterium smegmatis MC² 155 Wld-Typ, Mucobacterium smegmatis MC² 155 (NaiR) 6b, Mucobacterium smegmatis MC² 155 13a und E. coli DH5a besteht.

8. Pharmazeutische Zusammensetzung zum Inhibieren des Wachstums bakterieller Pathogene, wobei diese Zusammensetzung eine wirksame Menge der biologisch aktiven Fraktion CIM-109 oder teilweise gereinigten Extrakt oder lyophilisierten Extrakt von CIM-109, wie in Anspruch 5 beansprucht, umfasst.

9. Zusammensetzung wie in Anspruch 8 beansprucht, worin diese biologisch aktive Fraktion einzeln oder in Kombination verwendet wird.

10. Zusammensetzung wie in Anspruch 8 beansprucht, worin diese biologisch aktive Fraktion systemisch oder oral, bevorzugt oral, verabreicht werden kann.

11. Zusammensetzung wie in Anspruch 8 beansprucht, worin die biologisch aktive Fraktion dem Patienten in Kombination mit pharmazeutisch annehmbaren Additiven, Trägern, Verdünnungsmitteln, Lösungsmitteln, Filtern, Schmiermitteln, Exzipienten, Bindemitteln oder Stabilisatoren zu verabreichen ist.

12. Zusammensetzung wie in Anspruch 8 beansprucht, worin das Additiv, das verwendet wird, aus der Gruppe ausgewählt ist, die aus Zitronensäure, Kalziumcarbonat, Magnesiumhydroxydgel oder Gel oder Lactose besteht.

13. Zusammensetzung wie in Anspruch 8 beansprucht, worin die tägliche Dosis für den Menschen in einem Bereich von 500 mg bis 1000 mg liegt.

14. Zusammensetzung wie in Anspruch 8 beansprucht, worin die Menge der biologisch aktiven Fraktion in der Zusammensetzung in dem Bereich von 100 mg bis 500 mg liegt.

15. Pharmazeutische Zusammensetzung, die zur Behandlung von Fluorchinolon-resistenten bakteriellen Infektionen nützlich ist und Multi-Medikament-resistente bakterielle Infektionen inhibiert, einschließlich enterischer und systemischer Infektionen, wobei diese Zusammensetzung 10 bis 50 Gew.-% der biologisch aktiven Hexanfraktion aus dem Wurzelextrakt aus Vetivergras, 0,4 bis 1 Gew.-% Zitronensäure, 10 bis 20 Gew.-% Kalziumcarbonat, 10 bis 20 Gew.-% Magnesiumhydroxydgel, 20 bis 60 Gew.-% Lactose, ggf. zusammen mit anderen pharmazeutisch annehmbaren Zusatzstoffen, umfasst.

16. Pharmazeutische Zusammensetzung wie in Anspruch 15 beansprucht, welche ggf. mit Honig verbunden ist, indem die Bestandteile in Honig dispergiert werden.

17. Verwendung einer biologisch aktiven Hexanfraktion oder einer lyophilisierten biologisch aktiven Hexanfraktion aus den Wurzeln der Pflanze Vetivera zizanioides zur Herstellung eines Medikaments zur Behandlung bakterieller Infektionen, die durch Fluorchinolonresistente Bakterieninfektionen und Multi-Medikament-resistente Bakterieninfektionen verursacht werden, einschließlich enterischer und systemischer Infektionen.

18. Verwendung wie in Anspruch 17 beansprucht, wobei diese biologisch aktive Hexanfraktion einzeln oder in Kombination verwendet wird.

19. Verwendung wie in Anspruch 17 beansprucht, worin dieses Medikament zur systemischen oder oralen und bevorzugt zur oralen Verabreichung geeignet ist.

20. Verwendung wie in Anspruch 17 beansprucht, worin dieses Medikament weiter pharmazeutisch annehmbare Additive, Träger, Verdünnungsmittel, Lösungsmittel, Filter, Schmiermittel, Exzipienten, Bindemittel oder Stabilisatoren umfasst.

21. Verwendung wie in Anspruch 17 beansprucht, worin das Additiv, das verwendet wird, aus der Gruppe ausgewählt wird, die aus Zitronensäure, Kalziumcarbonat, Magnesiumhydroxydgel und/oder Gel und/oder Lactose besteht.

22. Verwendung wie in Anspruch 17 beansprucht, worin dieses Medikament für die tägliche Dosierung geeignet ist, die von 100 mg bis 500 mg reicht.

23. Verwendung wie in Anspruch 17 beansprucht, worin dieses Medikament für die bevorzugte Dosierung geeignet ist, die von 150 mg bis 250 mg reicht.

## Revendications

1. Procédé d'isolement d'une fraction bioactive comprenant les étapes de:
a) pulvérisation de la partie végétale de *Vetivera zizanioides,*
b) extraction de la poudre végétale de l'étape (a) par trempage dans un solvant organique aqueux protique pendant une période de 16-20 heures,
c) filtration de l'extrait de solvant organique de l'étape (b),
d) évaporation de l'extrait de l'étape (c) sous pression réduite pour éliminer le solvant organique en vue d'obtenir un extrait aqueux,
e) lyophilisation de l'extrait aqueux de l'étape (d) pour obtenir un extrait pulvérulent,
f) dissolution de l'extrait pulvérulent de l'étape (e) dans de l'acide citrique aqueux à 2 %,
g) extraction de la solution de l'étape (f) avec, successivement, du chloroforme, du n-butanol, du méthanol et finalement avec de l'acétone pour obtenir les extraits organiques respectifs et une solution aqueuse,
h) évaporation séparée des extraits organiques de l'étape (g) pour obtenir les résidus respectifs,
i) neutralisation de la solution aqueuse de l'étape (g) avec une solution d'ammoniac,
j) test de l'activité biologique des résidus obtenus dans l'étape (h) et de la solution neutralisée de l'étape (i) pour identifier le résidu de l'extrait méthanolique comme résidu bioactif,
k) macération du résidu de l'extrait méthanolique de l'étape (h) avec, successivement, de l'hexane, du chloroforme et de l'acétate d'éthyle,
l) test de l'activité biologique des fractions d'hexane, de chloroforme et d'acétate d'éthyle de l'étape (k) pour identifier les fractions d'hexane en tant que fraction bioactive,
m) purification du résidu de la fraction d'hexane de l'étape (l) sur une colonne de gel de silice en utilisant comme éluant de l'hexane et un mélange d'hexane-chloroforme avec une polarité croissante, et
n) évaporation de la fraction d'éluant hexane-chloroforme (1:1) obtenue à partir de l'étape (m) pour donner un résidu que l'on purifie par chromatographie sur couche mince pour obtenir la fraction bioactive voulue.

2. Procédé selon la revendication 1, dans lequel la fraction bioactive obtenue dans l'étape (m) est appelée CIM 109.

3. Procédé selon la revendication 1, dans lequel la partie végétale est choisie parmi les racines de *Vetivera zizanioides.*

4. Procédé selon la revendication 1, dans lequel, dans l'étape (b), le solvant organique aqueux protique est l'éthanol aqueux.

5. Fraction d'hexane bioactive CIM 109 issue de la plante *Vetivera zizanioides,* ayant une activité inhibitrice contre des pathogènes bactériens multirésistants aux médicaments, la fraction pouvant être obtenue à partir de l'étape (m) du procédé selon les revendications 1 à 4.

6. Fraction bioactive selon la revendication 5, ladite fraction bioactive inhibant le développement de pathogènes bactériens qui sont résistants à l'acide nalidixique, l'acide oxolinique, la sparfloxicine, la ciprofloxacine, la loméfloxicine et toute autre quinolone.

7. Fraction bioactive selon la revendication 6, la bactérie multirésistante aux médicaments étant choisie dans le groupe constitué par le genre *Mycobacterium* ou *Escherichia coli,* de préférence choisie dans le groupe constitué par *Mucobacterium smegmatis* MC² 155, *Pseudomonas aeruginosa, Bacillus subtilis* MTCC-121, *Mucobacterium smegmatis* MC² 155 de type sauvage, *Mucobacterium smegmatis* MC² 155 (NaiR) 6b, *Mucobacterium smegmatis* MC² 155 13a et *E. coli* DH5a.

8. Composition pharmaceutique destinée à l'inhibition du développement des pathogènes bactériens, ladite composition comprenant une quantité efficace de fraction bioactive CIM-109 ou d'un extrait partiellement purifié ou d'un extrait lyophilisé de CIM-109 selon la revendication 5.

9. Composition selon la revendication 8, où ladite fraction bioactive est utilisée isolément ou en combinaison.

10. Composition selon la revendication 8, où ladite fraction bioactive peut être administrée de façon systémique ou par voie orale, de préférence par voie orale.

11. Composition selon la revendication 8, où la fraction bioactive est destinée à être administrée au patient en combinaison avec des additifs, supports, diluant, solvant, filtre, lubrifiant, excipient, liant ou stabilisant pharmaceutiquement acceptables.

12. Composition selon la revendication 8, où l'additif utilisé est choisi dans un groupe constitué par l'acide citrique, le carbonate de calcium, un gel d'hydroxyde de magnésium ou un gel ou le lactose.

13. Composition selon la revendication 8, où la posologie journalière pour l'homme se situe dans la plage de 500 mg à 1 000 mg.

14. Composition selon la revendication 8, où la quantité de fraction bioactive dans la composition se situe dans le domaine de 100 mg à 500 mg.

15. Composition pharmaceutique utile pour le traitement d'infections bactériennes résistantes à la fluoroquinolone et l'inhibition d'infections bactériennes multirésistantes aux médicaments, comprenant des infections entériques et systémiques, ladite composition comprenant 10 à 50 % en poids de fraction d'hexane bioactive provenant d'un extrait de racines de vétiver, 0,4 à 1 % en poids d'acide citrique, 10 à 20 % en poids de carbonate de calcium, 10 à 20 % en poids de gel d'hydroxyde de magnésium, 20 à 60 % en poids de lactose et éventuellement d'autres additifs pharmaceutiquement acceptables.

16. Composition pharmaceutique selon la revendication 15, qui est éventuellement mélangée avec du miel par dispersion des constituants dans du miel.

17. Utilisation d'une fraction d'hexane bioactive ou d'une fraction d'hexane bioactive lyophilisée provenant des racines de la plante *Vetivera zizanioides* pour la préparation d'un médicament destiné au traitement d'infections bactériennes dues à des infections bactériennes résistantes à la fluoroquinolone et des infections bactériennes multirésistantes aux médicaments, comprenant des infections entériques et systémiques.

18. Utilisation selon la revendication 17, dans laquelle ladite fraction d'hexane bioactive est utilisée isolément ou en combinaison.

19. Utilisation selon la revendication 17, dans laquelle ledit médicament est approprié à une administration systémique ou orale, et de préférence à une administration orale.

20. Utilisation selon la revendication 17, dans laquelle ledit médicament comprend en outre des additifs, supports, diluant, solvant, filtre, lubrifiant, excipient, liant ou stabilisant pharmaceutiquement acceptables.

21. Utilisation selon la revendication 17, dans laquelle l'additif utilisé est choisi dans un groupe constitué par l'acide citrique, le carbonate de calcium, un gel d'hydroxyde de magnésium et/ou un gel et/ou le lactose.

22. Utilisation selon la revendication 17, dans laquelle ledit médicament est approprié à une posologie journalière allant de 100 mg à 500 mg.

23. Utilisation selon la revendication 17, dans laquelle ledit médicament est approprié à la posologie préférée allant de 150 mg à 250 mg.
